# FASCICULE DE BREVET EUROPEEN

(11) **EP 1 140 080 B1**
(45) Date de publication et mention de la délivrance du brevet: **06.10.2004**
(21) Numéro de dépôt: 99958307.3
(22) Date de dépôt: 13.12.1999
(51) Int. Cl.: A61K 31/425, A61P 27/16

(54) **UTILISATION DU RILUZOLE DANS LE TRAITEMENT DES TRAUMATISMES ACOUSTIQUES**
VERWENDUNG VON RILUZOL ZUR BEHANDLUNG VON AKUSTISCHEN TRAUMAZUSTÄNDEN
USE OF RILUZOLE FOR TREATING ACOUSTIC TRAUMAS

(30) Priorité: 15.12.1998 FR 9815834
(43) Date de publication de la demande: 10.10.2001
(73) Titulaire: Aventis Pharma S.A., 92160 Antony (FR)
(72) Inventeur: STUTZMANN, Jean-Marie, F-94440 Villecresnes (FR); RANDLE, John, Brookline, Ma 02445 (US)
(74) Mandataire: Rousseau, Pierrick Edouard
(86) Numéro de dépôt international: PCT/FR1999/003108
(87) Numéro de publication internationale: WO 2000/035447

(56) Documents cités:
- WO-A-94/13288
- WO-A-95/19170
- US-A- 5 624 945
- DATABASE CHEMABS [Online] CHEMICAL ABSTRACTS SERVICE, COLUMBUS, OHIO, US SHULMAN, ABRAHAM: "Neuroprotective drug therapy: a medical and pharmacological treatment for tinnitus control" retrieved from STN Database accession no. 131:13200 HCA XP002111218 & INT. TINNITUS J. (1998), VOLUME DATE 1997, 3(2), 77-93,
- DATABASE MEDLINE [Online] US NATIONAL LIBRARY OF MEDICINE (NLM), BETHESDA, MD, US KRETSCHMER B D ET AL: "Riluzole, a glutamate release inhibitor, and motor behavior." retrieved from STN Database accession no. 1998420451 XP002111219 & NAUNYN-SCHMIEDEBERGS ARCHIVES OF PHARMACOLOGY, (1998 AUG) 358 (2) 181-90.,
- EHRENBERGER K ET AL: "RECEPTOR PHARMACOLOGICAL MODELS FOR INNER EAR THERAPIES WITH EMPHASIS ON GLUTAMATE RECEPTORS: A SURVEY" ACTA OTO-LARYNGOLOGICA, vol. 115, no. 2, 1 mars 1995 (1995-03-01), pages 236-240, XP000609443

## Description

La présente invention concerne l'utilisation du riluzole ou un de ses sels pharmaceutiquement acceptables dans la prévention et/ou le traitement des traumatismes acoustiques et, notamment, des surdités et des acouphènes.

Le riluzole (2-amino-6-trifluorométhoxybenzothiazole) est commercialisé pour le traitement de la sclérose latérale amyotrophique. Ce composé est également utile comme anticonvulsivant, anxiolytique et hypnotique (EP50551), dans le traitement de la schizophrénie (EP305276), dans le traitement des troubles du sommeil et de la dépression (EP305277), dans le traitement des désordres cérébrovasculaires et comme anesthésique (EP282971), dans le traitement des traumatismes spinaux, crâniens ou crânio-spinaux (WO94/13288), comme radiorestaurateur (WO94/15600), dans le traitement de la maladie de Parkinson (WO94/15601), dans le traitement du neuro-sida (WO94/20103), dans le traitement des maladies mitochondriales (WO95/19170).

Il a maintenant été trouvé que le riluzole ou un de ses sels pharmaceutiquement acceptables peut aussi être utilisé dans la prévention et/ou le traitement des traumatismes acoustiques et, notamment, des acouphènes et des surdités et, en particulier, des surdités dues aux traumatismes sonores et des surdités liées à l'âge.

L'organe de Corti des mammifères possède 2 types de cellules : les cellules ciliées internes (CCI) et les cellules ciliées externes (CCE). Les CCE amplifient les vibrations de la membrane basilaire. Les CCI, véritables cellules sensorielles, transforment cette vibration mécanique en un message interprétable par le système nerveux central.

L'évaluation de l'état fonctionnel des cochlées de cobayes -ayant subi une surstimulation sonore intense (6kHz, 130 dB SPL pendant 15 minutes) montre des pertes auditives très importantes (de l'ordre de 80 dB). Une évaluation histologique de ces cochlées prélevées immédiatement après le traumatisme acoustique révèle non seulement des dommages mécaniques au niveau des cellules ciliées mais aussi un éclatement des dendrites des neurones auditifs primaires sous les CCI.

L'effet du riluzole à des doses croissantes a été étudié selon la technique de périfusion aiguë de la cochlée :
10 cobayes tricolores pesant 200 à 400 g sont anesthésiés par une injection intrapéritonéale d'uréthane (1,4 g/kg) puis trachéotomisés et ventilés artificiellement. Les conduits auditifs externes sont incisés pour la mise en place de barres d'oreilles dans un appareil de contention. Durant toute la durée de l'expérience, l'électrocardiogramme est contrôlé et la température centrale régulée au moyen d'une couverture chauffante à 38,5 ± 1°C. Les expérimentations sont réalisées dans une chambre insonorisée, anéchoïde et faradisée. L'accès à la cochlée est réalisé par abord ventral. Après ouverture de la bulle tympanique, 3 trous de 0,2 mm de diamètre sont fraisés manuellement dans le tour basal de la cochlée : le premier dans la rampe tympanique pour accueillir une pipette de perfusion, le second dans la rampe vestibulaire pour permettre l'écoulement hors de la cochlée des solutions perfusées et le troisième également dans la rampe vestibulaire pour recevoir une électrode d'enregistrement.

Les potentiels cochléaires enregistrés dans la rampe vestibulaire sont stockés puis filtrés numériquement pour dissocier le potentiel d'action composite (PAC), témoin de l'activité du nerf auditif et les potentiels de récepteurs provenant des cellules ciliées, à savoir le potentiel microphonique (PM) et le potentiel de sommation (PS). Un filtre passe-bas de 2,5 kHz est utilisé pour extraire le PAC dont l'amplitude est mesurée entre N 1 (1ère onde négative) et P1 (1ère onde positive) ainsi que le PS mesuré entre 0 et 5 ms. Un filtre passe-bande autour de 8 kHz (fréquence de la stimulation acoustique) est utlilisé pour extraire le PM (6-10 kHz, pente du filtre : 48 db/octave).

Après introduction de la pipette (environ 0,1 mm de diamètre à la poite) dans la rampe tympanique à l'aide d'un micromanipulateur, l'étanchéité étant assurée grâce à une boulle de colle placée près de la pointe, la périlymphe artificielle est perfusée à la vitesse de 2,5 µl/mn. Une seconde perfusion de périlymphe artificielle contenant 0,1 % de diméthilsulfoxyde est effectuée. Si cette seconde perfusion ne modifie pas les potentiels cochléaires, cette périlymphe sert de véhicule pour étudier l'effet de doses croissantes de riluzole sur les potentiels cochléaires. Ces potentiels cochléaires sont enregistrés immédiatement après chaque perfusion. La durée des perfusions est de 10 minutes.

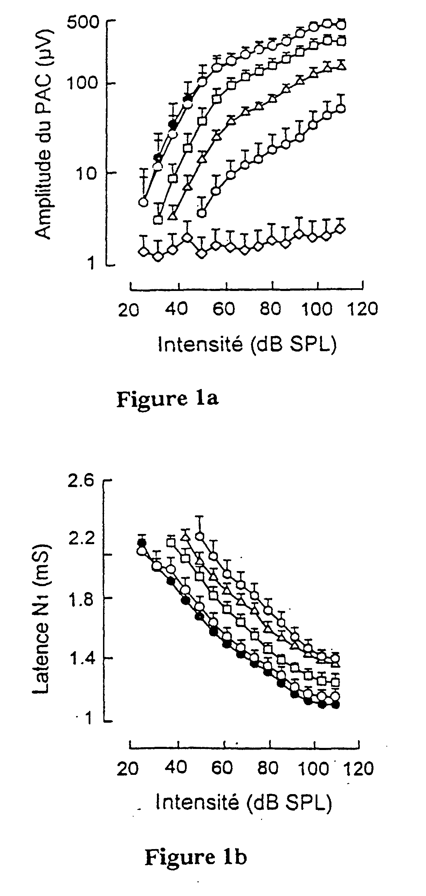

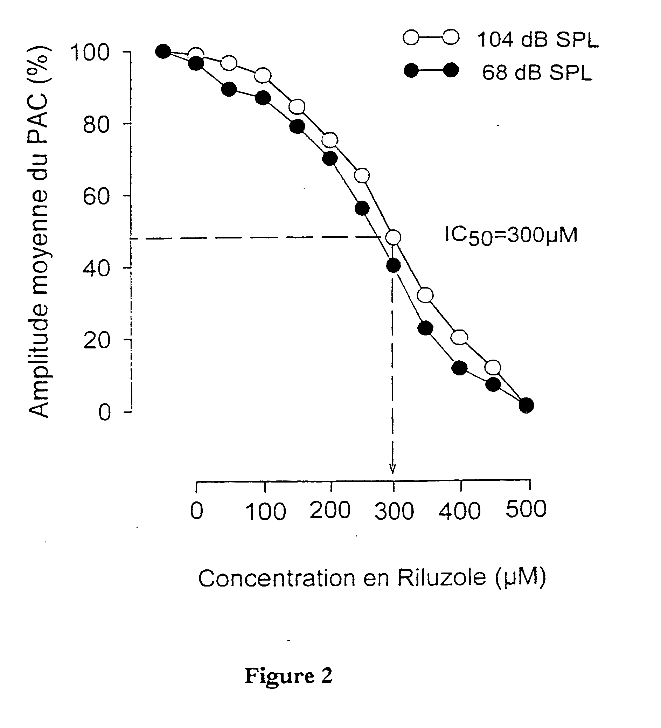

La perfusion de doses cumulatives et croissantes de riluzole (50 à 500 µM) diluées dans la même périlymphe contenant du diméthylsulfoxyde (0,1 %) provoque une réduction du potentiel d'action composite (PAC) du nerf auditif dépendant de la dose appliquée dans la cochlée et une augmentation de la latence N1 de ce potentiel (figures 1a et 1b, ronds pleins : la périlymphe seule, ronds vides : la périlymphe + diméthylsulfoxyde, carrés : la périlymphe contenant 250 µM de riluzole, triangles : la prérilymphe contenant 350 µM de riluzole, hexagones : la périlymphe contenant 450 µM de riluzole et losanges : la périlymphe contenant 500 µM de riluzole). L'IC50 est observée pour une dose de 300 µM (figure 2). Chez tous les animaux, une disparition totale du PAC est obtenue pour une dose de 500 µM.

L'effet protecteur sur le traumatisme acoustique a été testé sur 10 animaux pour lesquels une perfusion préalable de périlymphe artificielle est effectuée pendant 10 minutes. Après évaluation des seuils audiométriques, une seconde perfusion de 35 minutes soit de périlymphe artificielle seule (5 animaux : groupe témoin) soit de périlymphe contenant 500 µM de riluzole (5 animaux) est effectuée. Dix minutes après le début de la deuxième perfusion, une surstimulation sonore (6 kHz, 130 dB SPL) est appliquée durant 15 minutes. Enfin une troisième perfusion de périlymphe artificielle permet le rinçage de la cochlée. Les seuils audiométriques sont ensuite enregistrés. La perte auditive pour chaque fréquence induite par l'exposition sonore est définie comme la différence entre les seuils audiométriques enregistrés avant et après la surexposition sonore.

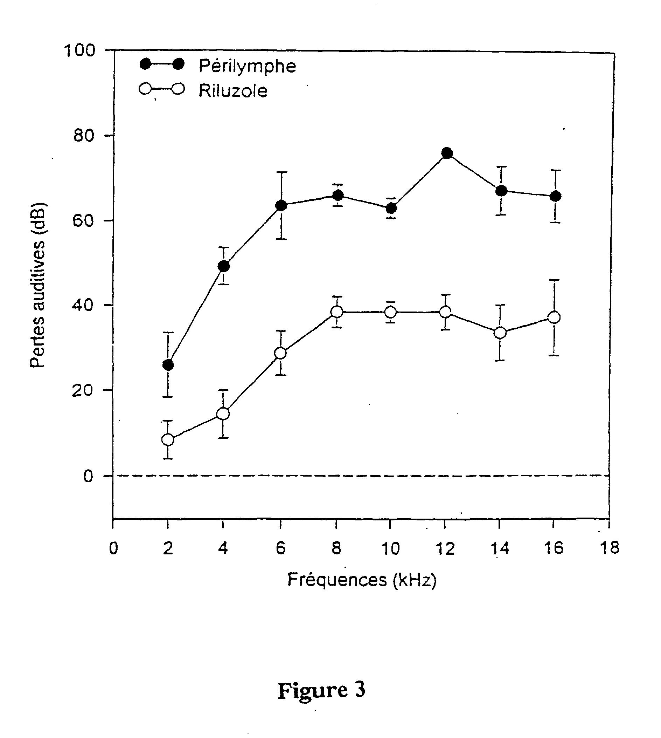

Le groupe témoin présente, pour des fréquences comprises entre 8 et 16 kHz, des pertes auditives beaucoup plus importantes que le groupe traité avec 500 µM de riluzole (figure 3, ronds pleins : périlymphe seule, ronds vides : périlymphe contenant 500 µM de riluzole).

L'étude histologique des cochlées en microscopie électronique montre chez le groupe témoin une destruction totale des terminaisons des fibres du nerf auditif et chez le groupe traité au riluzole, une innervation quasi normale.

Comme sels pharmaceutiquement acceptables du riluzole peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

Les médicaments sont constitués par au moins le riluzole sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 50 et 400 mg par jour par voie orale pour un adulte avec des doses unitaires allant de 25 à 200 mg de substance active.

D'une façon générale, le médecin déterminera la posologie appropriée en fonction de l'âge, du poids et de tous les autres facteurs propres au sujet à traiter.

Les exemples suivants illustrent des médicaments selon l'invention :

### Exemple A

On prépare, selon la technique habituelle, des comprimés dosés à 50 mg de produit actif ayant la composition suivante :
- Riluzole .............................................................................. 50 mg
- Mannitol ............................................................................. 64 mg
- Cellulose microcristalline .................................................. 50 mg
- Polyvidone excipient ........................................................ 12 mg
- Carboxyméthylamidon sodique........................................ 16 mg
- Talc ................................................................................... 4 mg
- Stéarate de magnésium .................................................. 2 mg
- Silice colloïdale anhydre ...............................................2 mg
- Mélange de méthylhydroxypropylcellulose, polyéthylèneglycol 6000, dioxyde de titane (72-3,5-24,5) q.s.p. 1 comprimé pelliculé terminé à 245 mg

### Exemple B

On prépare, selon la technique habituelle, des gélules dosées à 50 mg de produit actif ayant la composition suivante :
- Riluzole .............................................................................. 50 mg
- Cellulose ............................................................................ 18 mg
- Lactose ................................................................................ 55 mg
- Silice colloïdale................................................................... 1 mg
- Carboxyméthylamidon sodique ........................................ 10 mg
- Talc ................................................................................... 10 mg
- Stéarate de magnésium .................................................. 1 mg

### Exemple C

On prépare une solution injectable contenant 10 mg de produit actif ayant la composition suivante :
- Riluzole .............................................................................. 10 mg
- Acide benzoïque ................................................................. 80 mg
- Alcool benzylique ............................................................... 0,06 cm3
- Benzoate de sodium ............................................................ 80 mg
- Ethanol à 95 % .................................................................... 0,4 cm3
- Hydroxyde de sodium ............................................................... 24 mg
- Propylène glycol ................................................................. 1,6 cm3
- Eau ........................................................................... q.s.p. 4 cm3

L'invention concerne également le procédé de préparation de médicaments utiles dans la prévention et/ou le traitement des traumatismes acoustiques et, notamment, des acouphènes et des surdités et, en particulier, des surdités dues aux traumatismes sonores et des surdités liées à l'âge consistant à mélanger le riluzole ou les sels pharmaceutiquement acceptables de ce composé avec un ou plusieurs diluants et/ou adjuvants compatibles et pharmaceutiquement acceptables.

L'invention concerne également la méthode de prévention et/ou de traitement des traumatismes acoustiques et, notamment, des acouphènes et des surdités et, en particulier, des surdités dues aux traumatismes sonores et des surdités liées à l'âge consistant à administrer au patient du riluzole ou un de ses sels pharmaceutiquement acceptable.

## Revendications

1. Utilisation du riluzole ou un de ses sels pharmaceutiquement acceptables à la préparation de médicaments pour la prévention et/ou le traitement des traumatismes acoustiques.

2. Utilisation selon la revendication 1 pour la préparation de médicaments pour la prévention et/ou le traitement des acouphènes.

3. Utilisation selon la revendication 1 pour la préparation de médicaments pour la prévention et/ou le traitement des surdités.

4. Utilisation selon la revendication 1 pour la prévention et/ou le traitement des surdités dues aux traumatismes sonores.

5. Utilisation selon la revendication 1 pour la prévention et/ou le traitement des surdités liées à l'âge.

6. utilisation selon l'une des revendications 1 à 5 pour la préparation d'un médicament comprenant 25 à 200 mg de riluzole.

## Patentansprüche

1. Verwendung von Riluzol oder eines seiner pharmazeutisch verträglichen Salze zur Herstellung von Medikamenten zur Vorbeugung und/oder Behandlung akustischer Traumata.

2. Verwendung nach Anspruch 1 zur Herstellung von Medikamenten zur Vorbeugung und/oder Behandlung von Tinnitus.

3. Verwendung nach Anspruch 1 zur Herstellung von Medikamenten zur Vorbeugung und/oder Behandlung von Schwerhörigkeiten.

4. Verwendung nach Anspruch 1 zur Vorbeugung und/oder Behandlung von Schwerhörigkeiten aufgrund von Lärmtraumata.

5. Verwendung nach Anspruch 1 zur Vorbeugung und/oder Behandlung von altersbedingten Schwerhörigkeiten.

6. Verwendung nach einem der Ansprüche 1 bis 5 zur Herstellung eines Medikaments, das 25 bis 200 mg Riluzol umfasst.

## Claims

1. Use of riluzole or one of its pharmaceutically acceptable salts for the preparation of medicaments for the prevention and/or treatment of acoustic traumas.

2. Use according to claim 1, for the preparation of medicaments for the prevention and/or treatment of tinnitus.

3. Use according to claim 1, for the preparation of medicaments for the prevention and/or treatment of deafness.

4. Use according to claim 1, for the prevention and/or treatment of deafness caused by sound traumas.

5. Use according to claim 1, for the prevention and/or treatment of age-related deafness.

6. Use according to one of claims 1 to 5, for the preparation of a medicament comprising 25 to 200 mg of riluzole.
